# EUROPEAN PATENT APPLICATION

(11) **EP 4 000 641 A2**
(43) Date of publication of application: **25.05.2022**
(21) Application number: 21202955.7
(22) Date of filing: 15.10.2021
(51) Int. Cl.: A61L 2/04, B60H 1/00

(54) **USING TRANSPORT REFRIGERATION EQUIPMENT AS OVEN FOR PPE STERILIZATION**

(30) Priority: 24.11.2020 US 202063117781 P
(71) Applicant: Carrier Corporation, Palm Beach Gardens, FL 33418 (US)
(72) Inventor: MAYBURY, Daniel M., Syracuse, NY. 13221-4805 (US)
(74) Representative: Dehns

(57) **Abstract**

A transport refrigeration unit (TRU) system (301) is provided and includes a container (120) defining an interior (122) in which PPE (313) is storable, a TRU (320) to generate heated air for sterilizing the PPE (313) and an airflow system (330) disposed in the interior (122) and coupled to the TRU (320) to drive a flow of the heated air from the TRU (320), throughout the interior (122) and back to the TRU (320).

## Description

The present disclosure relates to transport refrigeration units (TRUs) and, in particular, to TRU equipment modified for use as a portable, large-capacity personal protective equipment (PPE) sterilization oven.

The COVID-19 crisis has revealed the need to sterilize PPE in large batches in a rapidly changing location and demand environment. While, temporary and/or portable facilities could provide that capability and would be very helpful to hospitals, clinics, and emergency service responders in terms of improving medical assistance response time, such temporary and/or portable facilities are not currently available.

According to a first aspect of the invention, a transport refrigeration unit (TRU) system is provided and includes a container defining an interior in which PPE is storable, a TRU to generate heated air for sterilizing the PPE and an airflow system disposed in the interior and coupled to the TRU to drive a flow of the heated air from the TRU, throughout the interior and back to the TRU.

The container may be a truck or trailer container or a standalone container.

The TRU may include a diesel engine driven TRU supported at least partially on the container.

The TRU may include a controller configured to operate a vapor compression cycle in a heating mode.

The TRU may include a controller configured to operate a vapor compression cycle in at least one of a cooling mode and a heating mode.

The TRU may be sized to maintain a temperature of 150°F [66°C] in the interior.

The TRU may be configured to generate the heated air at a temperature and mass flow rate sufficient to sterilize the PPE.

The TRU may be further configured to control a humidity of the interior for maintaining a sterile environment therein.

The airflow system may include one or more fans and blowers, an outflow duct extending away from an outlet of the TRU and an inflow duct extending toward an inlet of the TRU.

A storage system may be operably disposed in the interior and configured to receive the PPE in an unsterilized state and to store the PPE in the interior during sterilization.

At least one of a heating element may be disposed in the interior and configured to generate heat for sterilizing the PPE and a radiative element may be disposed in the interior and configured to emit radiation for sterilizing the PPE.

Power for sterilizing the PPE may be drawn from the TRU and one or more of a shore or standby power source, a generator power source and an external power source.

According to a second aspect of the invention, a method of operating a transport refrigeration unit (TRU) system is provided and includes operating a TRU in a heating mode to generate heated air for sterilizing PPE and driving a flow of the heated air from the TRU, throughout an interior of a container in which the PPE is stored and back to the TRU.

The operating of the TRU may include generating the heated air at a temperature and mass flow rate sufficient to sterilize the PPE.

The operating of the TRU may include controlling a humidity of the interior for maintaining a sterile environment therein.

The method may further include receiving the PPE in an unsterilized state in the interior and storing the PPE in the interior during sterilization.

The method may further include at least one of activating a heating element disposed in the interior to generate heat for sterilizing the PPE and activating a radiative element disposed in the interior to emit radiation for sterilizing the PPE.

According to a third aspect of the invention, a method of operating a transport refrigeration unit (TRU) system is provided and includes transporting a portable container to a location at which PPE is used, receiving the PPE in an unsterilized state, storing the PPE in an interior of the portable container, operating a TRU in a heating mode to generate heated air for sterilizing the PPE within the interior and driving a flow of the heated air from the TRU, throughout the interior and back to the TRU.

The operating of the TRU may include generating the heated air at a temperature and mass flow rate sufficient to sterilize the PPE.

The operating of the TRU may include controlling a humidity of the interior for maintaining a sterile environment therein.

The method may further include at least one of activating a heating element disposed in the interior to generate heat for sterilizing the PPE and activating a radiative element disposed in the interior to emit radiation for sterilizing the PPE.

Additional features and advantages are realized through the techniques of the present disclosure. Other embodiments and aspects of the disclosure are described in detail herein and are considered a part of the claimed technical concept. For a better understanding of the disclosure with the advantages and the features, refer to the description and to the drawings.

For a more complete understanding of this disclosure, reference is now made to the following brief description as a way of example only, and taken in connection with the accompanying drawings and detailed description, wherein like reference numerals represent like parts:
FIG. 1 is a side view of a truck/trailer with a TRU;
FIG. 2A is a schematic illustration of a vapor compression cycle of the TRU of FIG. 1 operating in a cooling mode;
FIG. 2B is a schematic illustration of a vapor compression cycle of the TRU of FIG. 1 operating in a heating mode;
FIG. 3 is a side schematic view of a TRU system for sterilizing PPE;
FIG. 4 is a flow diagram illustrating a method of operating a TRU system for sterilizing PPE; and
FIG. 5 is a flow diagram illustrating a method of operating a TRU system for sterilizing PPE.

As will be described below, software and hardware of TRU and container systems are adapted to provide heat required to sterilize PPE cargo per CDC and other guidance. In this way, readily available, mobile, large-batch sterilization ovens and drying chambers can be provided when and where they are needed to address the COVID-19 crisis and other similar situations.

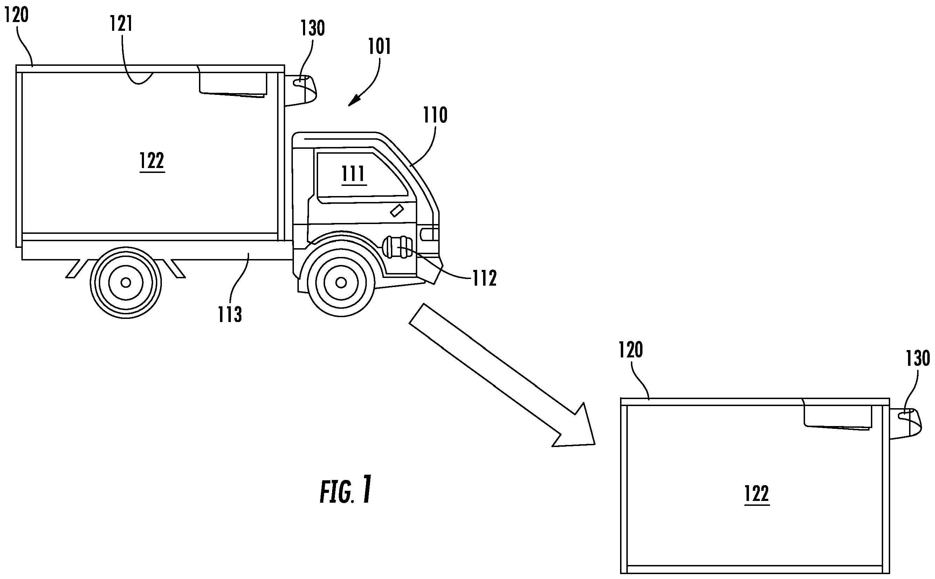

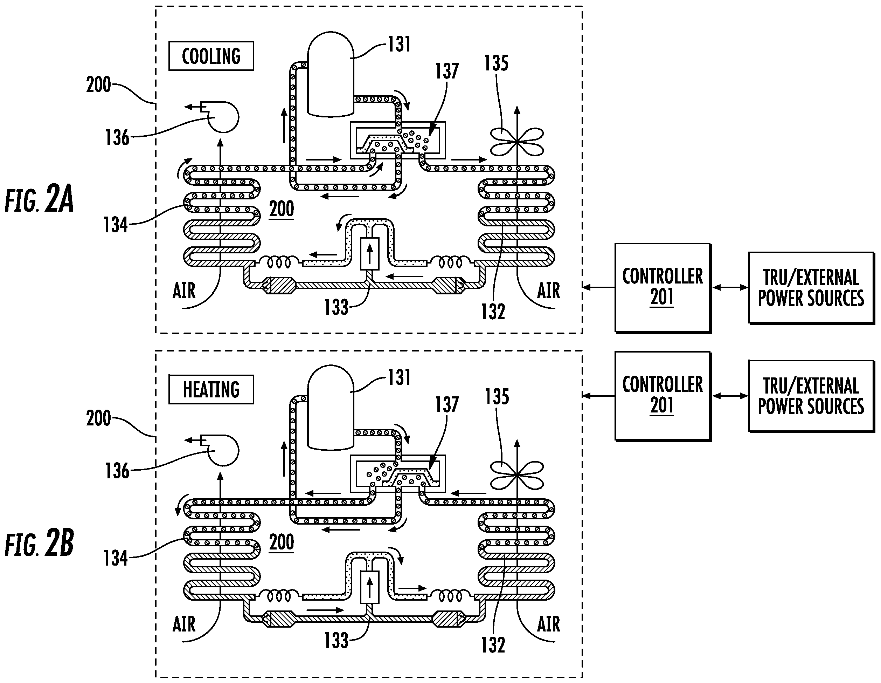

With reference to FIGS. 1 and 2, a truck or trailer 101 is provided and includes a vehicle portion 110, a trailer portion or container 120 and a TRU 130. The vehicle portion 110 includes a cabin 111, which is supportive of an engine 112 and which is configured to accommodate an operator and one or more passengers, and a bed frame 113 in the rear of the cabin 111. In some cases, the engine 112 can be provided as a diesel engine or another similar type of internal combustion engine, hybrid drive or electric drive. For clarity and brevity, the following description will relate to the case of the engine 112 being an internal combustion diesel engine. As shown, the trailer portion or container 120 may be portable and may be seated on the bed frame 113 or another land-based vehicle or on a flight or naval vessel. The trailer portion or container 120 can also be deployed into a standalone condition as shown in FIG. 1. In any case, the trailer portion or container 120 has a body 121 that is formed to define an interior 122 in which items being transported or shipped can be stored in a conditioned (heated or cooled) space. The TRU 130 is operably coupled directly or indirectly to the engine 112 and the body 121 of the trailer portion 120. Alternatively, the TRU can include an onboard engine. The TRU is configured to generate a cooling (or heating) airflow that can be directed into the interior 122 to control at least a temperature of the interior 122. In this way, in an exemplary case in which the items being transported are perishable and need to be refrigerated, the TRU 130 can maintain the interior 122 in a refrigerated state even during hot days. Conversely, in an exemplary case in which the items being transported are perishable and need to be heated, the TRU 130 can maintain the interior 122 in a heated state even during cold days.

As shown in FIGS. 2A and 2B, the TRU 130 can be operated in at least one of a cooling mode and a heating mode (e.g., by changing the direction in which the refrigerant flows in the vapor compression cycle 200). For example, the refrigerant may flow clockwise when in a cooling mode (FIG. 2A), and counterclockwise when in a heating mode (FIG. 2B). In certain instances, the TRU 130 includes a controller 201 operably coupled to the vapor compression cycle 200 to control the operation of the TRU 130 (i.e., whether the TRU 130 is operating in the heating mode or the cooling mode). As shown in FIGS. 2A and 2B, the vapor compression cycle 200 may include a compressor 131, a first heat exchanger 132, an expansion valve assembly 133 including metering valves, a second heat exchanger 134, a fan (or a blower) 135, a blower (or a fan) 136 and a reversing valve 137. The compressor 131 is provided to both move and increase the pressure of the refrigerant flowing through the vapor compression cycle 200. The reversing valve 137 is provided to change the direction the refrigerant flows. The expansion valve 133 is provided to regulate the flow of the refrigerant. The first heat exchanger 132 and the second heat exchanger 134 are provided to transfer heat either to or from the refrigerant flowing through the vapor compression cycle 200 (which may be dependent on the direction in which the refrigerant is flowing).

When operated in the cooling mode by the controller 201, the reversing valve 137 is set in a first position as shown in FIG. 2A. As shown, this may cause the refrigerant to flow in a clockwise manner. The compressor 131 compresses the refrigerant and outputs the compressed refrigerant to the first heat exchanger 132. Within the first heat exchanger 132, the compressed refrigerant (e.g., which may be in a vapor state) is condensed into a liquid through thermal interaction with air blown by the fan 135. The refrigerant is then directed through the expansion valve assembly 133 to the second heat exchanger 134. The refrigerant flows through coils of the second heat exchanger 134 while the blower 136 directs air over the coils to transfer heat from the air to the refrigerant. This cooled air is directed into the interior 122 of the container 120. As shown in FIG. 2A, after transferring heat from the air in the second heat exchanger 134, the refrigerant is transferred to the compressor 131, completing the cycle.

When operated in the heating mode by the controller 201, the reversing valve 137 is set in a second position as shown in FIG. 2B. As shown, this may cause the refrigerant to flow in a counterclockwise manner. The compressor 131 compresses the refrigerant and outputs the compressed refrigerant to the second heat exchanger 134. The refrigerant flows through the coils of the second heat exchanger 134 while the blower 136 directs air over the coils to transfer heat to the air from the refrigerant. This heated air is directed into the interior 122 of the container 120. As shown in FIG. 2B, after transferring heat to the air in the second heat exchanger 134, the refrigerant is transferred through the expansion valve assembly 133 to the first heat exchanger 132. The refrigerant flows through the coils of the first heat exchanger 132 while the fan 135 blows air over the coils to transfer heat to the refrigerant. As shown in FIG. 2B, after transferring heat from the air to the refrigerant in the first heat exchanger 132, the refrigerant is transferred to the compressor 131, completing the cycle.

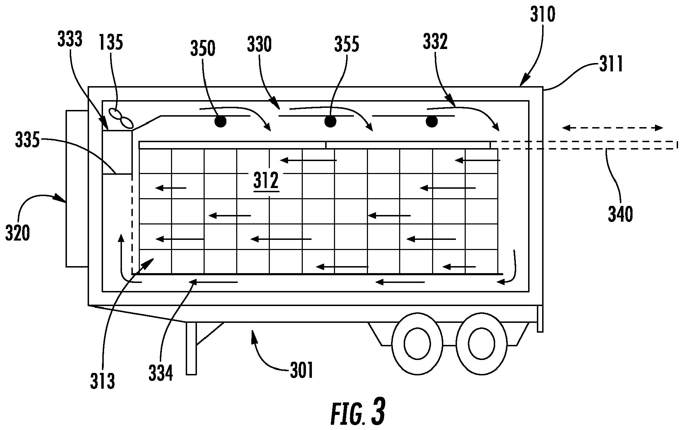

With continued reference to FIGS. 1, 2A and 2B and with additional reference to FIG. 3, a TRU system 301 is provided. The TRU system 301 includes a container 310 that includes a body 311 formed to define an interior 312 in which PPE 313 is storable, a TRU 320 to generate heated air (when operating in the heating mode, as described above) for sterilizing the PPE 313, and an airflow system 330. The container 310 can be a truck or trailer container, such as the trailer portion 120 of FIG. 1, and can be portable and transported to a location where PPE is used. The TRU 320 can be a diesel engine driven TRU that is supported at least partially on the body 311 of the container 310. The TRU 320 can include a vapor compression cycle 200, such as the vapor compression cycle 200 of FIG. 2, and a controller 201. As described above, the controller 201 may be operably coupled to the vapor compression cycle 200 to control the direction in which the refrigerant flows (i.e., to switch between the cooling mode and the heating mode). The airflow system 330 is disposed in the interior 312 and is coupled to the TRU 320 to drive a flow of the heated air from the TRU 320, throughout the interior 312 and back to the TRU 320.

It should be appreciated that the TRU 320 can be sized and modified such that the TRU 320 is configured to generate the heated air at a temperature and mass flow rate which is sufficient to sterilize the PPE 313 within the interior 312. For example, the TRU 320 may be sized to achieve a temperature within the interior 312 of about 150°F [66°C] (of course, it is understood that faster sterilizations times may be achieved at T ≥ 150°F [66°C]). In addition, the TRU 320 may also be sized and modified such that the TRU 320 is further configured to control a humidity of the interior 312 for maintaining a sterile environment therein. For example, the TRU 320 may be sized to maintain the humidity in the interior 312 at about 85%RH in combination with the -149/150°F [65/66°C] temperature setting. The TRU 320 may also be sized such that there is sufficient flow rate to circulate the conditioned air evenly throughout the interior.

As shown in FIG. 3, the airflow system 330 can include one or more fans or blowers, such as the fan 135 and the blower 136 of FIG. 2, an outflow duct 332 extending away from an outlet 333 of the TRU 320 with outflow duct outlets arranged in one or more various arrangements and an inflow duct 334 extending toward an inlet 335 of the TRU 320.

In accordance with further embodiments, the TRU system 301 can also include one or more of a storage system 340 (e.g., shelving, drawers, etc.), a heating element 350 and a radiative element 355. The storage system 340 can be operably disposed in the interior 312 and configured to extend and retract to thereby receive the PPE 313 in an unsterilized state and to store the PPE 313 in the interior 312 during sterilization. The heating element 350 can be disposed in the interior 312 and configured to generate heat for sterilizing the PPE 313. The heating element 350 can include or be provided as a resistive heating element and can draw power from the TRU 320. Alternatively or additionally, the heating element 350 can draw power from the TRU 320 and one or more of a shore or standby power source, a generator power source and an external power source. In these or other cases, the power can be drawn to the heating element 350 through the TRU 320 from the one or more of the shore or standby power source, the generator power source and the external power source. The radiative element 355 can include or be provided as a light generating element (i.e., a UV light generating element) and can draw power from the TRU 320. Alternatively or additionally, the radiative element 355 can draw power from the TRU 320 and one or more of the shore or standby power source, the generator power source and the external power source. As above, in these or other cases, the power can be drawn to the radiative element 355 through the TRU 320 from the one or more of the shore or standby power source, the generator power source and the external power source.

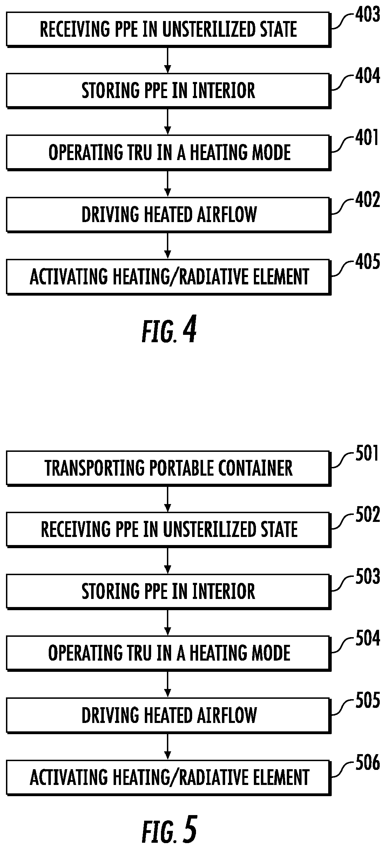

With reference to FIG. 4, a method of operating a TRU system, such as the TRU system 301 of FIG. 3, is provided. As shown in FIG. 4, the method may include step 403 for receiving PPE in an unsterilized state, step 404 for storing PPE in the interior of a container, step 401 for operating the TRU in a heating mode to generate heated air for sterilizing PPE 401, and step 402 for driving a flow of the heated air from the TRU throughout the interior of the container in which the PPE is stored. In addition, the method may include step 405 for as activating one or more of a heating element and a radiative element disposed in the interior of the container to generate additional heat for sterilizing the PPE.

With reference to FIG. 5, a method of operating a TRU system, such as the TRU system 301 of FIG. 3, is provided. As shown in FIG. 5, the method can include step 501 for transporting a portable container to a location at which PPE is used, step 502 for receiving the PPE in an unsterilized state, step 503 for storing the PPE in an interior of the portable container, step 504 for operating a TRU in a heating mode to generate heated air for sterilizing the PPE within the interior 504, and step 505 for driving a flow of the heated air from the TRU throughout the interior of the container. In addition, the method may include step 506 for activating one or more of a heating element and a radiative element disposed in the interior of the container to generate additional heat for sterilizing the PPE.

In accordance with further embodiments, the methods of FIGS. 4 and 5 can include a determination operation in which it is determined whether there is sufficient power available in the TRU to sterilize the PPE. If so, power is only drawn from the TRU. If not, additional power required to sterilize the PPE can be drawn from the TRU and one or more of the shore or standby power source, the generator power source and the external power source. That is, the TRU will supply heat and power for sterilization methods and would be modified and reconfigured to put out as much heat as possible for sterilization, and perhaps extra power for additional, external heating elements and other sterilization methods. The TRU can be modified to supply an outlet that is powered by the TRU or by the external (shore or genset or other) power that the TRU plugs into. A transformer could be provided to transform multi-phase high voltage to lower, single phase voltage to power the outlet as needed for potential sterilization equipment.

It is to be understood that the description provided above relates to cases in which PPE is sterilized in the interior 122 of the trailer portion or container 120 (see FIG. 1) while the trailer portion or container 120 is static or parked and while the trailer portion or container 120 is mobile. In the mobile case, sterilized PPE can be delivered to a location by the truck or trailer 101 so that it can be usable immediately (e.g., on-demand).

Technical effects and benefits of the present disclosure are the provision of mobile solutions for and rapid response to the need for large scale sterilization of PPE.

The corresponding structures, materials, acts, and equivalents of all means or step-plus function elements in the claims below are intended to include any structure, material, or act for performing the function in combination with other claimed elements as specifically claimed. The description of the present invention has been presented for purposes of illustration and description, but is not intended to be exhaustive or limited to the technical concepts in the form disclosed. Many modifications and variations will be apparent to those of ordinary skill in the art without departing from the scope of the invention as set out in the claims. The embodiments were chosen and described in order to best explain the principles of the invention and the practical application, and to enable others of ordinary skill in the art to understand the invention for various embodiments with various modifications as are suited to the particular use contemplated.

While the preferred embodiments to the invention have been described, it will be understood that those skilled in the art, both now and in the future, may make various improvements and enhancements which fall within the scope of the claims which follow. These claims should be construed to maintain the proper protection for the invention first described.

## Claims

1. A transport refrigeration unit (TRU) system (301), comprising:
a container (120) defining an interior (122) in which PPE (313) is storable;
a TRU (320) to generate heated air for sterilizing the PPE (313); and
an airflow system (330) disposed in the interior (122) and coupled to the TRU (320) to drive a flow of the heated air from the TRU (320), throughout the interior (122) and back to the TRU (320).

2. The TRU system (301) according to claim 1, wherein the container (120) is a truck or trailer container or a standalone container.

3. The TRU system (301) according to claim 1 or 2, wherein the TRU (320) comprises a diesel engine driven TRU supported at least partially on the container (120).

4. The TRU system (301) according to claim 1, 2 or 3, wherein the TRU (320) comprises a controller (201) configured to operate a vapor compression cycle (200) in at least one of a heating mode and a cooling mode, and/or configured to generate the heated air at a temperature and mass flow rate sufficient to sterilize the PPE (313).

5. The TRU system (301) according any preceding claims, wherein the TRU (320) is sized to maintain a temperature of about 150°F [66°C] in the interior (122), and/or the TRU (320) is further configured to control a humidity of the interior (122) for maintaining a sterile environment therein.

6. The TRU system (301) according to any preceding claims, wherein the airflow system (330) comprises:
one or more fans and blowers;
an outflow duct (332) extending away from an outlet (333) of the TRU (320); and
an inflow duct (334) extending toward an inlet (335) of the TRU (320).

7. The TRU system (301) according to any preceding claims, further comprising a storage system (340) operably disposed in the interior (122) and configured to receive the PPE (313) in an unsterilized state and to store the PPE (313) in the interior (122) during sterilization.

8. The TRU system (301) according to any preceding claims, further comprising at least one of:
a heating element disposed in the interior (122) and configured to generate heat for sterilizing the PPE (313); and
a radiative element disposed in the interior (122) and configured to emit radiation for sterilizing the PPE (313).

9. The TRU system (301) according to any preceding claims, wherein power for sterilizing the PPE (313) is drawn from the TRU (320) and one or more of a shore or standby power source, a generator power source and an external power source.

10. A method of operating a transport refrigeration unit (TRU) system (301), the method comprising:
operating (401; 504) a TRU (320) in a heating mode to generate heated air for sterilizing PPE (313); and
driving (402; 505) a flow of the heated air from the TRU (320), throughout an interior (122) of a container (120) in which the PPE (313) is stored.

11. The method according to claim 10, wherein the operating of the TRU (320) comprises generating the heated air at a temperature and mass flow rate sufficient to sterilize the PPE (313).

12. The method according to claim 10 or 11, wherein the operating of the TRU (320) comprises controlling a humidity of the interior (122) for maintaining a sterile environment therein.

13. The method according to claim 10, 11 or 12, further comprising:
receiving (403; 502) the PPE (313) in an unsterilized state in the interior (122); and
storing (404; 503) the PPE (313) in the interior (122) during sterilization.

14. The method according to any of claims 10 to 13, further comprising at least one of:
activating (405; 506) a heating element (350) disposed in the interior (122) to generate heat for sterilizing the PPE (313); and
activating (405; 506) a radiative element (355) disposed in the interior (122) to emit radiation for sterilizing the PPE (313).

15. A method according to any of claims 10 to 14, the method comprising:
transporting a portable container to a location at which PPE (313) is used;
receiving the PPE (313) in an unsterilized state, and ;
storing the PPE (313) in an interior of the portable container;
